(19) Europäisches Patentamt
European Patent Office
Office européen des brevets

(11) Publication number: **0 354 303 A1**

(12) **EUROPEAN PATENT APPLICATION**

(21) Application number: 89107998.0

(22) Date of filing: 03.05.89

(51) Int. Cl.4: C07C 317/32 , C07C 323/37 , C07D 295/14 , C07D 333/24 , A61K 31/155

The title of the invention has been amended (Guidelines for Examination in the EPO, A-III, 7.3).

(30) Priority: 19.05.88 US 195930

(43) Date of publication of application: 14.02.90 Bulletin 90/07

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Lin, Yang-I
35 Constitution Drive
Tappan New York 10983(US)
Inventor: Wang, Bosco Shang
10 Parkview Road
Cranbury New Jersey 08512(US)
Inventor: Ruszala-Mallon, Veronica M.
395 North Little Tor Road
New City New York 10956(US)
Inventor: Bitha, Panayota
287 Treetop Circle
Nanuet New York 10954(US)
Inventor: Fields, Thomas Lynn
66 Amory Avenue
Pearl River New York 10965(US)

(74) Representative: Wächtershäuser, Günter, Dr.
Tal 29
D-8000 München 2(DE)

(54) **Amidines of diphenyl sulfones, their derivatives and their use.**

(57) This disclosure describes novel diphenyl sulfones, diphenyl sulfides, and diphenyl sulfoxides, which possess activity as modulators of the mammalian immune response system.

EP 0 354 303 A1

# MODULATORS OF THE IMMUNE RESPONSE SYSTEM

## CROSS REFERENCE TO RELATED APPLICATION

This application is a continuation-in-part of our co-pending application Serial No. 07/195,930, filed May 19, 1988.

## BRIEF SUMMARY OF THE INVENTION

This invention relates to new organic compounds and, more particularly, is concerned with novel diphenyl sulfides, diphenyl sulfoxides and diphenyl sulfones which may be represented by the following structural formula:

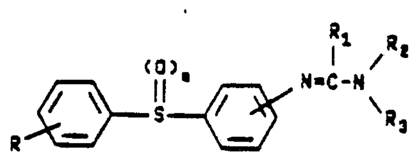

(I)

wherein m is 0, 1 or 2;
R is hydrogen, halogen, amino or a moiety of the formula:

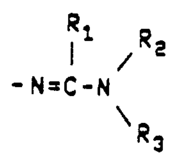

,

wherein $R_1$ is hydrogen, alkyl($C_1$-$C_4$), pyridine, phenyl,

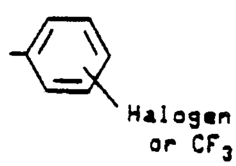

,

or thienyl; with the proviso that when $R_1$ is hydrogen, $R_2$ and $R_3$ may not be hydrogen or lower alkyl or taken together with the associated nitrogen, $R_2$ and $R_3$ may not be pyrrolidino, piperidino or morpholino and when $R_1$ is phenyl, $R_2$ and $R_3$ may not be hydrogen;
$R_2$ is hydrogen or alkyl ($C_1$-$C_4$);
$R_3$ is hydrogen, alkyl ($C_1$-$C_4$), phenyl or N,N-dimethylaniline;
$R_1$ and $R_2$ taken together may be $-(CH_2)_n-$ where n is an integer from 2 to 4 or $R_1$ and $R_2$ taken together may form a moiety of the formula:

- N - $H_2CH_2$-
  |
  $CH_3$

where $R_3$ is methyl;
$R_2$ and $R_3$ taken together with the associated N(itrogen) may be pyrrolidino, piperidino, morpholino, thiomorpholino, 4-methylpiperazino, 3-azabicyclo[3.2.2]nonyl, 2-pyridylpiperazino or 4-fluorophenylpiperazino; and the pharmaceutical acceptable salts thereof.

The novel compounds of the present invention are obtainable as light yellow to colorless crystalline

materials having characteristic melting points and absorption spectra.

The organic bases of this invention form non-toxic acid-addition salts with a variety of pharmacologically acceptable organic and inorganic salt-forming reagents. Thus, acid-addition salts, formed by admixture of the organic free base with one or two equivalents of an acid, suitably in a neutral solvent, are formed with such acids as sulfuric, phosphoric, hydrochloric, hydrobromic, sulfamic, citric, lactic, tartaric, acetic, benzoic, gluconic ascorbic, and the like. For purposes of this invention, the free bases are equivalent to their non-toxic acid addition salts.

The novel compounds of the present invention are active as modulators of the immune response systems in mammals as demonstrated in experimental animals hereinafter. Accordingly, this invention is concerned with a method of modulating the immune response system in warm-blooded animals employing compounds of formula (I); wherein m is 0, 1 or 2; R is hydrogen, halogen, amino or a moiety of the formula:

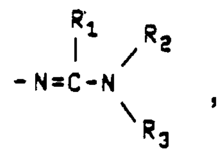,

$R_1$ is hydrogen, alkyl ($C_1$-$C_4$), pyridine, phenyl,

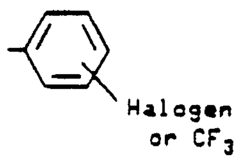,

or thienyl;
$R_2$ is hydrogen or alkyl ($C_1$-$C_4$);
$R_3$ is hydrogen, alkyl ($C_1$-$C_4$), phenyl or N,N-dimethylaniline;
$R_1$ and $R_2$ taken together may be $-(CH_2)_n-$ where n is an integer from 2 to 4 or $R_1$ and $R_2$ taken together form a moiety of the formula:

- N – $H_2CH_2$-
$CH_3$

where $R_3$ is methyl;
or $R_2$ and $R_3$ taken together with the associated nitrogen is pyrrolidino, piperidino, morpholino, thiomorpholino, 4-methylpiperazino, 3-aza-bicyclo [3.2.2] nonyl, 2-pyridylpiperazino, or 4-fluorophenylpiperazino; with pharmaceutical compositions of matter containing these compounds; and with chemical methods for their preparation.

## DESCRIPTION OF THE INVENTION

The novel compounds of the present invention may be readily prepared in accordance with the following reaction scheme:

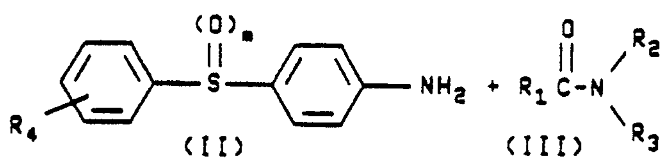
(II) (III)

wherein $R_4$ is hydrogen, halogen or amino and $R_1$, $R_2$, $R_3$ and m are as hereinbefore defined.

In accordance with the above reaction scheme, an appropriately substituted amide (III) is treated with phosphorus oxychloride in dry acetonitrile at 0° C. to about 10° C. and then stirred for an hour or more until

the temperature rises to room temperature. Then, a hemimolar amount of 4-aminophenyl sulfone (4′,4-sulfonyldianiline)(II) or an equimolar amount of 4′-(4-halophenylsulfonyl)aniline (II) is added to the reaction mixture and stirring is continued first at room temperature for a few hours and then at 60° C. for 2-16 hours. The reaction mixture is then poured into water and basified and the precipitated product is collected by filtration and recrystallized.

The use of immunomodulators and chemotherapeutic adjuvants constitutes a new therapeutic approach to the treatment of immune deficiencies and cancer and is based on the concept that there are distinctive antigens in or on most tumor cells (embryonal or transplantation antigens), that distinguish them from normal host cells.

A majority of tumor immunologists favor the view that potentially malignant cells constantly arise but because of their "foreignness" they are normally eliminated by a competent humoral and cellular immune system. Occasionally however, tumor cells escape this immune surveillance and continue to reproduce and cancer results. The reason for the failure of the normally efficient immune surveillance mechanisms is not fully understood but it is thought that the immune system becomes less effective with increasing age. It is also depressed in certain genetic immuno-deficiency diseases, in various bacterial, fungal or viral infections, and in patients undergoing immuno-suppressive therapy. The growth of the neoplasm itself, as well as the various therapeutic modalities designed to treat the disease, e.g., cytotoxic chemotherapy and radiation, leads to a still greater depression of host resistance and results in an increased susceptibility to both exogenous and endogenous infections and perhaps accounts for re-initiation of tumor growth and metastasis which frequently follows treatment-induced tumor remission.

This relationship between the immune system and tumor growth has been supported by experimental studies and observations in the clinical setting. Law, L. W., Nature, 205, 672-673 (1965) observed a marked increase in the incidence of polyoma tumors in thymectomized immunocompromised mice. In the clinical setting, Gatti, R. A. and Good, R. A., Cancer Genetics Lynch, H. T. (ed.) Charles Thomas, Springfield, IL (1976) observed that patients with immunodeficiencies appeared to have an increased susceptibility to neoplastic diseases and suggested that immune function had a relationship to malignancy. Eilber, F. R. and Morton, D. L., Cancer, 25, 362-367 (1970) also reported that cancer patients with an inability to develop a delayed type hypersensitivity response to skin test antigens generally had a poor prognosis. These observations imply that tumor initiation and progressive growth of malignant cells may be monitored and controlled by the host's immune network possibly via T cells, macrophages, or natural killer cells. On the other hand, the surveillance hypothesis has been questioned as a result of the apparent unrestricted growth of certain tumors in animals, Schwartz, R. S., New Engl. J. Med., 293. 181-184 (1975). The reasons for tumor cells escaping from surveillance are not completely understood, but several possibilities have been postulated including: (a) failure to recognize tumor antigens; (b) inability to mount adequate immune responses in order to destroy tumors; and (c) induction of immunodepression by oncogenic agents, blocking factors and/or suppressor cells. The ability to overcome these limitations via modulation of the immune defense system becomes important not only for the prophylaxis but also for the therapy of cancers.

If depression of the immune system can result in the growth of malignancies, regulation of any facet of the immune response may help the host to eliminate residual cancer cells. Therefore, it is desirable to search for chemical agents (i.e., immunomodulators) capable of restoring and stimulating host immune defense mechanisms in order to overcome the deficiencies which account for susceptibility to disease and failure to eradicate the cancer. Such immuno-modulating agents would likely be incapable of arresting the growth of a large tumor but their clinical utility would derive from their capacity to enhance normal immune surveillance mechanisms in patients whose tumor burden has been reduced by surgical, radiotherapeutic or chemotherapeutic methods.

Although a large number of agents including both biological and chemical substances have been tested for potential immunoenhancing activity against tumors, only a few have received critical evaluation in clinical trials [Oldham, R. K., J. Natl. Cancer Inst., 70, 789-796 (1983)]. Whether or not these candidates become therapeutically useful remains to be determined.

Nevertheless, experimental studies in animals have demonstrated the antitumor potential of a number of immunoregulants including live organisms of Bacillus Calmett-Guerin (BCG), Lamm, K. L., et al., Immunotherapy of Human Cancer, Terry and Rosenberg (eds.), 315-322, NY (1982), Corynebacterium parvum(C. parvum), Bast, R. C., et al., Cancer Res., 42 1395-1401 (1983), thymosin, Dillman, R. O., et al., J. Biol. Resp. Modif., 1, 35-41 (1982), interferons, Louie, A., et al., Blood, 58, 717-718 (1981), monoclonal antibodies, Ritz, J. and Schlossman, S.F., Blood, 59, 1-11 (1982), interleukins, Lotze, M.T., et al., J. Biol. Resp. Modif., 3, 475-482 (1984), polynucleotides, and the anthelmintic drug, levamisole.

These substances have been shown to stimulate cellular immunity and to produce tumor regression.

Some successes have been claimed in early clinical trials with BCG against malignant melanoma and acute leukemia, and with levamisole against lung cancer and breast cancer. Although the antitumor effects produced by these agents have been promising, significant therapeutic benefits have yet to be realized. Since this is a new therapeutic approach, new drugs and methods of treatment must receive careful clinical evaluation in order to reveal their full potential.

Modern research is directed to the discovery of a drug similar to but more potent than, known immunomodulators such as levamisole that would be effective in the eradication of tumor cells when used in conjunction with standard therapeutic measures. Stimulators of host resistance may be detected in animal models that can, in fact, detect both immunostimulators and anticancer agents. In one experimental animal model, mice are put in a condition simulating immunodepression common to cancer patients. This is accomplished by infecting mice with a leukemia virus which produces both leukemia and a disease-related immunodepression. Effective drugs are recognized by their ability to restore or enhance the antibody response in the experimental mice, or to inhibit tumor progression.

Such agents that would be capable of modulating the immune system would not only be useful in inhibiting tumor progression, but would be extremely useful as adjuncts in the chemotherapeutic or radiotherapeutic treatment of cancer and other related conditions. A major side effect associated with such chemotherapeutic or radiotherapeutic therapy is myelosuppression which then limits the dose of drug used and/or the frequency of treatment. Deaths due to chemo or irradiation associated myelosuppression are generally due to hemorrhage or sepsis. The hemorrhagic deaths are a result of thrombocytopenia, a drastic decrease in the number of platelets. Septic deaths are a result of neutropenia, a severe drop in neutrophils, the cell which plays a major role in recovery from bacterial infections. Septic deaths occur even when patients are treated with antibiotics.

However, it is known that with many cancers, the outcome may be better if one could use a more aggressive therapeutic approach by either increasing the dose or frequency of chemo or radiotherapy. Thus, a method for protecting the bone marrow from cytotoxic agents or accelerating the recovery of bone marrow cells following these regimens may allow for such aggressive therapy. One such approach to overcoming the hematologic toxicity associated with cancer therapies is autologous bone marrow transplantation to accelerate recovery of hematopoietic cells. More recently, several factors have been identified which are known stimulators of hematopoietic cell growth. These colony stimulating factors (CSFs) act on a variety of bone marrow progenitor cells to accelerate their differentiation into mature, active cell populations. Several of these factors have been cloned and are currently in trials in cancer patients who have undergone a variety of chemotherapeutic regimens. Results show that these agents, in particular, G-CSF (granulocyte colony stimulating factor) and GM-CSF (granulocyte-macrophage colony stimulating factor), could stimulate a sustained rise in neutrophil counts thus reducing the period of neutropenia after administration of a cytotoxic agent. A reduction in the number of days of neutropenia may prove beneficial not only in terms of hospital costs (by reducing the need for inpatient care) but ultimately in alleviating the morbidity and mortality associated with cancer therapy.

However, there are several drawbacks to treatment of chemotherapy associated myelosuppression with the recombinant colony stimulating factors. For one, there have been studies which indicate toxicities associated with GM-CSF therapy itself. Secondly, since the recombinant products are protein in nature, they have a relatively short half life when given intravenously. Thus, there is a need for continuous I.V. infusion to maintain therapeutically active levels. Thirdly, current studies are pointing to the need for combination therapy with various cytokines in order to obtain maximal responsiveness in bone marrow cell recovery. Compounds such as those described in this invention have several advantages over the recombinant CSFs. The primary advantage lies in the oral effectiveness of these immunomodulators, thus eliminating the need for continuous I.V. infusion and longer hospital stays. Secondly, the cost of manufacturing a synthetic compound is much less than that of a recombinant protein, thus translating into reduced cost for the patient. Thirdly, orally active synthetic compounds such as those described here are very stable compared with the recombinant proteins which are easily degraded once administered I.V.. Thus, a single oral dose of the synthetic compounds results in therapeutic effects comparable to a 14 day continuous infusion regimen with the recombinant CSFs.

And finally, one additional advantage of the synthetic compounds over the CSFs lies in the multiple cytokine induction by the synthetics, in particular, IL-1 and IL-2. Thus, one can achieve in a single oral dose with the synthetic compounds what multiple cytokines may do with repeated dosing.

The results as shown here suggest that these compounds may be an alternative to the CSFs to allow more aggressive therapy with cytotoxic drugs whose dose limiting toxicity is severe myelosuppression. Effective drugs in this series are recognized by their ability to accelerate recovery of bone marrow colony forming cells following 5-FU therapy and by stimulation of a colony forming activity in the serum of mice

which allows for the proliferation of normal bone marrow cells in culture. Their radioprotective properties are measured by their ability to enhance the number of colony forming cells in the spleen of treated mice (endogenous CFU-S). Their ability to augment production of IL-1, a cytokine which acts synergistically with the CSFs, is measured by the ability of macrophage supernatants of treated mice to promote growth of IL-1 dependent cells in culture. Also, the ability of the compounds to augment production of IL-2, a cytokine which plays a major role in immunoregulation, is measured by the ability of supernatants obtained from Con-A stimulated splenocytes to promote growth of the CTLL-2 IL-2 dependent cell line.

In accordance with the present invention, the orally active compounds of this invention have been shown to be capable of modifying the reactivity of certain immune cell populations which may effect the growth of a tumor. Evidence of this affect on immune cell populations by the test compounds was seen with macrophages, which have long been recognized as an immune cell important in controlling the development and spread of neoplasms, Macknass, G.B., The Macrophage in Neoplasia, M. A. Fink (ed.), 3-13, Academic Press, NY (1976). The test compounds apparently were able to activate these cells in vivo to inhibit the growth of tumor cells in cultures. These "activated" macrophages were detectable in peritoneal exudates of treated mice on day 4 after receiving a single oral dose of the test compound. (Table I). Macrophages and lymphocytes from treated mice release significantly more IL-1 and IL-2-like factors in culture than did the control counterparts. (Tables II and III). Sera from treated mice also possessed more colony stimulating factor than those from normal mice. (Table V). Mice treated with the test compounds also respond better to foreign antigens such as sheep red blood cells (SRBC) as shown by their ability to produce higher levels of antibody to this foreign protein. (Table IV). These experiments suggest that compounds of this invention may enhance the host's ability to respond to other foreign proteins such as those expressed on the surface of tumor cells.

The test compounds also accelerate recovery of bone marrow cells following chemotherapy in much the same way as the recombinant CSFs. (Figures 1-4). They are also radioprotective as shown in the endogeneous CFU-S assay. (Table VI).

The activity of the compounds of this invention as immunomodulators has been demonstrated in a series of procedures described below.

## Materials and Methods

### Animals, tumors and Reagents

C57BL/6 (B16, H-$2^b$), DBA/2 (D2, H-$2^d$), Balb/c (H-$2^d$), BDF1 (H-$2^d$) and CD2F1 (H-$2^d$) mice were obtained from Cumberland View Farms, Clinton, TN. C3H/HeJ (C3H, H-$2^k$) mice were obtained from the Jackson Laboratories, Bar Harbor, ME. All animals used were 6-10 weeks of age.

P815 mastocytoma was maintained in its syngeneic host. The appropriate tumor cells were used as targets in cytotoxicity assays. An interleukin-2 (IL-2) dependent cell line, designated CTLL-2, was maintained in culture in the presence of rat IL-2.

Hank's balanced salt solution (HBSS), RPMI 1640 medium, horse serum, fetal calf serum (FCS), L-glutamine, penicillin, streptomycin, Dulbecco's phosphate buffered saline (D-PSB), and N-2-hydroxyethylpiperazine-N′-2-ethansulfonic acid (HEPES) were obtained from Grand Island Biological Company, Grand Island, NY. Gentamicin was obtained from Upjohn Co., Kalamazoo MI; thioglycolate medium and lipopolysaccharide (LPS, E. coli 0128:B12) from DIFCO Laboratories, Detroit, Mi. $^{51}$Cr-sodium chromate ($^{51}$Cr, specific activity = 300-500 Ci/g) and methyl-$^3$H-thymidine ($^3$HTdR, specific activity = 20 Ci/mmol) were obtained from New England Nuclear, Boston, MA.

## I. Activation of Tumoricidal Macrophages

### Preparation of Murine Macrophages

Mice were injected intraperitoneally (IP) with 1 ml of thioglycolate medium and the peritoneal exudate (PE) cells were harvested 4-5 days later by washing their peritoneal cavities with HBSS containing 10 units/ml of heparin. PE cells were washed 3 times with HBSS and suspended in RPMI 1640 medium

containing 10% FCS, 100 U/ml of penicillin, 100 μg/ml of gentami cin, 2mM L-glutamine and 10mM HEPES. The cells were dispensed into flat-bottom wells of 96-well culture plates (Costar, Cambridge, MA) and incubated at 37°C for 2 hours. Non-adherent PE cells were removed by repeated and vigorous washings with HBSS and the majority of the remaining adherent cells (>95%) resembled macrophages morphologically and exhibited Mac-1 surface antigens as detected by fluorescence staining technique. These cells were also functionally active in ingesting latex particles.

Assay for Macrophage Mediated Tumor Cytostasis

P815 tumor cells obtained from D2 mice were washed twice with HBSS and suspended in 10% FCS-complete medium. Five x $10^3$ P815 cells were added to culture wells containing B6 macrophages and the E:T ratios were determined by the numbers of PE cells added initially to each well. Unless otherwise noted, culture plates were incubated for 2 days. Target cells in each well were pulsed with 0.5 Ci $^2$HTdR for the final 4 hours and harvested by a cell harvester. The amound of $^3$HTdR incorporated into target cells was determined in a liquid scintillation counter. The mean cpm were obtained from triplicate cultures with results being presented as percent cytostasis calculated by the following formula: % cytostasis = (A-B)/A x 100 where A = cpm of cultures containing normal control macrophages; and B = cpm of cultures containing experimental macrophages obtained from compound-treated mice.

Table I

| Activation of Tumoricidal Macrophages | | | | | | |
|---|---|---|---|---|---|---|
| | % Activation Test No. | | | | | |
| Compound | 1* | 2** | 3** | 4** | 5** | 6** |
| N′,N‴-(Sulfonyldi-p-phenylene)bis[N,N-diethylbutyramidine] | 59.5 | 39.9 | 44.2 | 39.3 | 58.1 | 55.8 |
| N′,N‴-(Sulfonyldi-p-phenylene)bis[N,N-dimethylpropionamidine] | 58.5 | 15.8 | 0 | 25.1 | NT | NT |
| 2,2′-[Sulfonylbis (p-phenylenenitrilo)]bis[1-methylpyrrolidine] | 13.4 | 21.5 | 10.0 | 29.4 | NT | NT |
| N′-[4-[(4-Fluoro phenyl)sulfonyl]phenyl]-N,N-dimethyl propanimidamide, monohydrochloride | NT | NT | NT | NT | 58.5 | NT |
| N′-[4-[(4-Fluoro phenyl)sulfonyl] phenyl]-N,N-dimethyl ethanimidamide | NT | NT | NT | NT | NT | 52.6 |
| N′-[4-[(4-Fluoro phenyl)sulfonyl] phenyl]-N,N-dimethyl ethanimidamide, monohydrohloride | NT | NT | NT | NT | NT | 34.8 |
| N′,N‴-(Sulfonyldi-p-phenylene)bis-[N,N-diethylacetamidine | 59.0 | 61.8 | 0 | 16.5 | NT | NT |
| N′,N‴-(Sulfonyldi-1,4-phenylene)bis[N,N-dimethylacetamidine] | 43.4 | 42.6 | 9.2 | 14.7 | NT | NT |
| NT = Not tested | | | | | | |

* - in vitro activation
** = in vivo activation

## II. Production of IL-1

IL-1 Assay

Groups of C57B1/6 mice were treated orally with immunomodulator at doses of 25, 100 or 200 mg/Kg. Four days layer peritoneal exudate cells (PEC) were collected and 1 x $10^5$ cells were plated in RPMi-1640 medium containing 5% fetal calf serum (FCS). After 2 hours incubation at 37°C the non-adherent cells were washed off and the adherent cells were incubated for 24 hours in RPMi medium with 5% FCS with or

without Lipopolysaccharide (10 ug/ml). The following day the supernatants were collected and assayed for IL-1 on either thymocytes or on the D10 IL-1 dependent cell line. The cultures were incubated for either 3 days (thymocytes) or 1 day (D-10) and then pulsed with $^3$H-TdR using 0.5 $\mu$Ci/well. The cells were harvested and the number of counts per minute (CPM) was determined using a Beckman scintillation counter.

**Table II**
**Results Of IL-1 Assays**

| Compound | Fold Increase Above Normals | | | Average | Evaluation |
|---|---|---|---|---|---|
| | Exp. #1 | Exp. #2 | Exp. #3 | | |
| N′,N‴-(Sulfonyl-di-p-phenylene)bis-[N,N-diethylethanimidamide] | 1.4 | 2.5 | 2.6 | 2.1 | ++ |
| N′,N‴-(Sulfonyl-di-p-phenylene)bis-[N,N-diethylbutyramidine] | 1.6 | 2.5 | 5.3 | 3.1 | +++ |
| N′,N‴-(Sulfonyl-di-p-phenylene)bis-[N,N-dimethylpropionamidine] | 3.1 | 2.5 | 10.6 | 5.4 | +++ |
| 2,2′-[Sulfonylbis-(p-phenylenenitrilo)]-bis[1-methylpyrrolidine] | 1.2 | 0.0 | 9.3 | 3.5 | +++ |
| 3-[1-[[4-[(4-Fluorophenyl)sulfonyl]phenyl]imino]ethyl]-3-azabicyclo[3.2.2]nonane | 2.8 | 1.0 | 0.0 | 1.2 | + |
| 3,3′-[Sulfonylbis(4,1-phenylenenitriloethylidyne)]bis-3-azabicyclo[3.2.2]-nonane | N.D. | 2.5 | 0.0 | 1.25 | + |
| N′-[4-[(4-Fluorophenyl)sulfonyl]phenyl]-N,N-dimethylpropanimidamide | N.D. | 2.3 | - | 0.0 | - |

**Table II (contd.)**
**Results Of IL-1 Assays**

| Compound | Fold Increase Above Normals | | | Average | Evaluation |
|---|---|---|---|---|---|
| | Exp. #1 | Exp. #2 | Exp. #3 | | |
| N′-[4-[(4-Fluorophenyl)-sulfonyl]phenyl]-N,N-dimethylpropanimidamide, monohydrochloride | 1.4 | 1.9 | 0.0 | 1.1 | + |
| N′-[4-[(4-Fluorophenyl)-sulfonyl]phenyl]-N,N-dimethylethanimidamide | 1.8 | 2.5 | 0.0 | 1.43 | + |
| N′-[4-[(4-Fluorophenyl)-sulfonyl]phenyl-N,N-dimethylethanimidamide, monohydrochloride | 0.0 | 2.3 | 0.0 | 0.76 | ± |

* = Elevated levels of IL-1-like activity in the absence of LPS
N.D. = Not Done
\- = Negative
± = Less than 1 fold increase over normals
\+ = Between 1 and 2 fold increase over normals
++ = Between 2 and 3 fold increase over normals
+++ = Greater than 3 fold increase over normals

III Production of IL-2

Preparation of Mouse Lymphocytes

Spleens were removed from mice and single-cell suspensions were prepared by teasing the spleens apart and rinsing through No. 40 and No. 80 stainless steel mesh screens. Erythrocytes were lysed by a 3-minute exposure to a 0.83% Tris-ammonium chloride solution. Cells were washed 3 times with HBSS and finally suspended in complete RPMI 1640 culture medium consisting of 5% FCS, 2mM L-glutamine, 5 x $10^{-5}$M 2-mercaptoethanol, 10 mM HEPES, 100 U/ml of penicillin, and 100 mg/ml of streptomycin. Cells were counted in a hemocytometer and the viability of test cells was always greater than 98% as judged by trypan blue dye exclusion.

Preparation of Various Soluble Factors and Their Assays

IL-2 was made by stimulating $10^7$ B6 splenocytes with 1 mg Con A in 1 ml medium. Supernatants were harvested 2 days later and residual Con A was removed by Sephadex G-50 absorption or a-methyl mannoside inactivation. IL-2 activity was tested in the thymocyte proliferation assay described above. Additionally, test samples were added to cultures of lymphoblasts prepared by a three-day Con A stimulation or to IL-2 dependent CTLL-2 cells. The proliferation of these indicator cells was measured 24 hours later by $^3$HTdR incorporation.

Enhancement and Restoration of the Production of IL-2-like Factor in Normal Mice

Lymphocytes prepared from normal mice or mice treated with 100 mg/kg of test compound were stimulated with Con A for 2 days. Culture supernatants were harvested and tested for putative IL-2 activity in 3 proliferation assays using thymocytes, lymphoblasts and CTLL-2 cells as indicators. Although supernatants from normal lymphocytes supported the growth of all indicator cells, lymphocytes from treated animals apparently produced more IL-2 as indicated by a greater degree of cell proliferation in all three test systems.

Table III

| Production of Il-2 | | | | | |
|---|---|---|---|---|---|
| | CPM Test No. | | | | |
| Compound | 1 | 2 | 3 | 4 | Evaluation |
| N′,N‴-(Sulfonyldi-p-phenylene)bis[N,N-diethylbutyramidine] | 10979 | NT | NT | NT | + |
| N′,N‴-(Sulfonyldi-p-phenylene)bis[N,N-dimethylpropionamidine] | NT | 15952 | 46617 | 129910 | + + |
| 2,2′-[Sulfonylbis (p-phenylenenitrilo)]bis[1-methylpyrrolidine] | NT | 17010 | NT | NT | + + + |
| N′-[4-[(4-Fluorophenyl)sulfonyl]phenyl]-N,N-dimethylethanimidamide | 4230 | NT | NT | NT | + |
| N′-[4-[(4-Fluorophenyl)sulfonyl]phenyl]-N,N-dimethylethanimidamide, monohydrochloride | 19945 | NT | NT | NT | + + |
| N′N‴-Sulfonyldi-p-phenylene)bis[N,N-diethylacetamidine] | NT | 17051 | NT | NT | + + + |
| Control | 3152 | 1920 | 23878 | 90570 | |

NT = Not tested

Control = Untreated normal mice

\+ = 1 to 3 fold increase above normal controls

\+ + = 4 to 7 fold increase above normal controls

\+ + + = 8 to 10 fold increase above normal controls

## IV. Anti-SRBC Antibody Assay

Compounds were given orally on day 0 at doses of 200 or 600 mg/kg. 0.1 ml of a 10% suspension of sheep RBC (Red Blood Cell) was given IP on day +4. Ten days later splenocytes were obtained from these mice and either 4 x $10^6$ or 2 x $10^6$ splenocytes in 50 ml were mixed with 50 ml of a 15% suspension of SRBC containing guinea pig complement (1:4 dilution). 100 ml of this mixture was placed onto a slide culture and incubated for 30 to 45 minutes at 37°C. The number of antibody forming cells was determined by counting the number of visible plaques formed.

Table IV

| Production of Anti-SRBC Antibody | | | |
|---|---|---|---|
| | PFC/$10^7$ splenocytes Test No. | | |
| Compound | 1 | 2 | Evaluation |
| N′,N‴-(Sulfonyldi-p-phenylene)bis[N,N-diethylbutyramidine] | NT | 1765 | + + + |
| N′,N‴-(Sulfonyldi-p-phenylene)bis[N,N-dimethylpropionamidine | NT | 726 | NS |
| N′,-[4-[(4-Fluorophenyl) Sulfonyl]phenyl]-N,N-dimethylpropanimidamide | 1933 | NT | + + |
| N′-[4-[(4-Fluorophenyl)sulfonyl]phenyl]-N,N-dimethylethanimidamide | 2138 | NT | + + |
| N′-[4-[(4-Fluorophenyl)sulfonyl]phenyl]-N,N-dimethylethanimidamide, monohydrochloride | 1167 | NT | NS |
| Control | 1260 | 830 | - |

NT = Not tested

NS = Not significant

+ + = 1.5 fold increase above normal controls

+ + + = 2.0 fold increase above normal controls

Control = Untreated normal mice

## V. Colony Stimulating Factor Production

### Preparation of Mouse Bone Marrow Cells

Mouse femurs were removed and the marrow cells collected by aspiration through a 23 gauge needle and dispensed into 5 ml of RPMI 1640 culture medium. Aliquots were diluted with a 2% solution of acetic acid containing 0.2% crystal violet and the number of nucleated cells per femur was determined by direct count in a hemocytometer.

### Effect on CSF Induction in Mice

Mouse sera were assayed for colony stimulating factor (CSF) in cultures of normal bone marrow cells. CSF was tested in a system in which 5 x $10^4$ normal marrow cells were incubated with 10% test samples in each well of 96-well culture plates for 3 days. Proliferation of these cells was determined by $^3$HTdR incorporation.

**Table V**
**Results of CSF Proliferation Assay**

| Compound | % Increase Above Normal Serum Expt. # 1 | 2 | 3 | 4 | 5 | Average | Evaluation |
|---|---|---|---|---|---|---|---|
| N-[4[(4-Fluorophenyl) sulfonyl]-phenyl]acetamide * | 35.1 | N.D. | 50.0 | 22.0 | 42.5 | 37.5 | + |
| N',N'''-(Sulfonyldi-p-phenylene)bis[N,N-diethylethanimidamide] | 23.1 | 104.0 | 0.0 | 4.8 | 11.5 | 28.6 | ± |
| N',N'''-(Sulfonyldi-p-phenylene)bis[N,N-diethylbutyramidine] | 14.4 | 122.0 | 211.0 | 22.4 | 112.5 | 96.0 | +++ |
| N',N'''-(Sulfonyldi-p-phenylene)bis[N,N-dimethylpropionamidine] | 0.0 | 164.0 | 150.0 | 66.0 | 75.0 | 91.0 | +++ |
| 2,2'-[Sulfonylbis(p-phenylenenitrilo]bis-[1-methylpyrrolidine] | 25.6 | 210.0 | 116.0 | 96.0 | 71.0 | 103.0 | +++ |
| 3-[1-[[4-[(4-Fluorophenyl)sulfonyl]phenyl] imino]ethyl]-3-azabicyclo [3.2.2]nonane | 96.0 | N.D. | 0.0 | N.D. | 48.0 | 48.0 | + |
| 3,3'-[Sulfonylbis(4,1-phenylenenitriloethylidyne)]bis-3-azabicyclo-[3.2.2]nonane | 115.0 | 151.0 | 55.0 | 52.0 | 85.0 | 92.0 | +++ |
| N'-[4-[(4-Fluoro phenyl)sulfonyl]phenyl]-N,N-dimethylpropanimidamide | 33.3 | 142.0 | N.D. | 42.0 | N.D. | 72.0 | ++ |

**Table V contd.**

**Results of CSF Proliferation Assay**

| Compound | % Increase Above Normal Serum Expt. # 1 | 2 | 3 | 4 | 5 | Average | Evaluation |
|---|---|---|---|---|---|---|---|
| N'-[4-[(4-Fluorophenyl)sulfonyl]-phenyl]-N,N-dimethylpropanimidamide, monohydrochloride | 108.0 | 130.0 | N.D. | 29.0 | N.D. | 89.0 | ++ |

N.D. = Not Done
\- = Negative
± = Less than 30% increase above normals
\+ = 30 to 50% increase above normals
++ = 50 to 90% increase above normals
+++ = Greater than 90% increase above normals
* = This compound is disclosed in U.S. Patent No. 4,532,349

VI. Endogenous CFU-S Assay for Radioprotection

Groups of Balb/c mice were given one oral dose of immunomodulator at 25, 100, or 200 mg/kg on day-1 relative to 600 rads of whole body gamma irradiation. Two weeks after irradiation the mice were sacrificed and the spleens removed and fixed in Bouin's fixative prior to counting colonies. The values are expressed as the number of colonies/spleen.

Table VI

| Endogenous CFU-S Assay for Radioprotection | |
|---|---|
| | Number of Colonies/Spleen |
| 600 Rad Control | 35 |
| 600 Rads + N′, N‴-(Sulfonyldi- p -phenylene)bis[ N , N -dimethylpropionamidine] | |
| 400 mg/kg | 35 |
| 200 mg/kg | 73 |
| 100 mg/kg | 46 |
| 25 mg/kg | 52 |

VII. CFU-C Assay to Measure Acceleration of Myeloid Cell Recovery Following 5-FU Therapy

Groups of C3H/Hej mice were treated I.P. with 5-Fluorouracil (5-FU) (150 mg/kg). Four days later they were given one oral dose of immunomodulator at 25, 100, or 200 mg/kg. Three days later the mice were sacrificed and bone marrow cells collected by aspiration through a 23 gauge needle and dispensed into 5 ml of RPMi-1640 culture medium. Aliquots were diluted with a 0.2% solution of acetic acid in PBS, containing 0.2% crystal violet. The number of nucleated cells per femur was determined by direct count in a hemocytometer. The cells were plated in agarose containing medium supplemented with an exogenous source of CSF (50 u/plate of GM-CSF). After 7 days in culture the number of colonies consisting of 50 or more cells are counted and the values expressed as CFU-C per 100,000 cells. The results are shown in Figures 1-4.

| Legend for Figure 1: | |
|---|---|
| G1. = | Normals |
| G2. = | 5-Fluorouracil |
| G3. = | 5-Fluorouracil + control, N-[4-[(4-fluorophenyl) sulfonyl]phenyl]acetamide, (disclosed in U.S. Pat. No. 4,532,349) at 100mg/kg. |
| G4. = | 5-Fluorouracil + N′,N‴-(Sulfonyldi-p-phenylene)bis[N,N-d ethylbutyramidine] at 100 mg/kg |
| G5. = | 5-Fluorouracil + N′,N‴-(Sulfonyldi-p-phenylene)bis[N,N-d methylpropionamidine] at 200 mg/kg |
| G6. = | 5-Fluorouracil + 2,2′-[Sulfonylbis(p-phenylenenitrilo)]bis[1.methylpyrrolidine] at 200 mg/kg |

| Legend for Figure 2: | |
|---|---|
| G1 = | Normal |
| G2 = | 5-Fluorouracil |
| G3 = | 5-Fluorouracil + control, N-[4-[(4-Fluorophenyl)sulfonyl]phenyl]acetamide (U.S. 4,532,349) at 100 mg/kg. |
| G4 = | 5-Fluorouracil + N,N‴-(Sulfonyldi-p-phenylene)bis[N,N-diethylacetami e] at 25 mg/kg |

| Legend for Figure 3: | |
|---|---|
| G1 = | Normals |
| G2 = | 5-Fluorouracil |
| G3 = | 5-Fluorouracil + control, N-[4-[(4-Fluorophenyl)sulfonyl]phenyl]acetamide (U.S. 4,532,349) at 100 mg/kg. |
| G4 = | 5-Fluorouracil + 3-[1-[[4-[(4-Fluorophenyl)sulfonyl]sulfonyl]imino]ethyl]3-azabicyclo[3.2.2]nonane at 200 mg/kg |
| G5 = | 5-Fluorouracil + 3,3′-[Sulfonylbis(4,1-phenylenenitriloethylidyne)bis-3-azabicyclo[3.2.2]nonane at 100 mg/kg |

| Legend for Figure 4 | |
|---|---|
| G1 = | Normal |
| G2 = | 5-Fluorouracil |
| G3 = | 5-Fluorouracil + control, N,-[4-[(4-Fluorophenyl)sulfonyl]phenyl]acetamide (U.S. 4,532,349) at 100 mg/kg. |
| G4 = | 5-Fluorouracil + N′-[4-[(4-Fluorophenyl)sulfonyl]phenyl]-N,N-dimethylethnimidamide at 6.2 mg/kg |
| G5 = | 5-Fluorouracil + N′-[4-[(4-Fluorophenyl)sulfonyl]phenyl]-N,N-dimethylethanimidamide, monohydrochloride at 25 mg/kg |
| G6 = | 5-Fluorouracil + N′-[4-[(4-Fluorophenyl)sulfonyl]phenyl]N,N-dimethylpropanimidamide at 200 mg/kg |
| G7 = | 5-Fluorouracil + N′-[4-[(4-Fluorophenyl)sulfonyl]phenyl]N,N-dimethylpropanimidamide, monohydrochloride at 200 mg/kg |

The compounds of the present invention are effective as immunomodulators (that is, they modulate the immune response) when administered in amounts ranging from about 5 mg to about 400 mg/kg of body

weight per day. A preferred dosage regimen for optimum results would be from about 25 mg to about 500 mg/kg of body weight per day. This dosage regimen may be adjusted to provide the optimum therapeutic response. For example, several divided doses may be administered daily or the dose may be proportionally reduced as indicated by the exigencies of the therapeutic situation. A practical advantage of this invention is that the active compounds may be administered in any convenient manner such as the oral or buccal routes.

The compounds of the present invention may be orally administered, for example, with an inert diluent or with an assimilable edible carrier, or they may be enclosed in hard or soft shell gelatin capsules, or they may be compressed into tablets. For oral therapeutic administration, the active compounds may be incorporated with excipients and used in the form of tablets, troches, capsules, elixirs, suspensions, syrups, wafers and the like. Such compositions and preparations should contain at least 0.5% of active compound. The percentage of the compositions and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 500 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen or cherry flavoring. When the dosage unit form is a capsule, it may contain, in addition to materials of the above type, a liquid carrier such as fatty oil. Various other materials may be present as coatings or to otherwise modify the physical form of the dosage unit. For instance, tablets, pills of capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed.

The invention will be described in greater detail in conjunction with the following non-limiting examples.

## Example 1

### N′,N‴-(Sulfonyldi-p-phenylene)bis[N,N-diethylbutyramidine]

To a solution of 21.4 g of N,N-diethylbutyramide in 100 ml of dry acetonitrile was added 18.4 g of phosphorus oxychloride at a temperature of 5° to 10°C. This mixture was stirred at room temperature for 1 hour then 12.4 g of 4,4′-diaminodiphenyl sulfone was added. Stirring was continued at room temperature for 4 hours, then at 60°C for 2 hours, poured into 1 liter of ice-water and basifed with 5 N sodium hydroxide. The solid was collected, washed with water and recrystallized from chloroform/hexane, giving 24.2 g of the desired product as colorless crystals, mp 136-138°C.

## Example 2

### N′,N‴-(Sulfonyldi-p-phenylene)bis[N,N-dimethylpropionamidine

To a solution of 15.2 g of N,N-dimethylpropionamide in 100 ml of dry acetonitrile was added 18.4 g of phosphorus oxychloride at a temperature of 5° to 10°C. This mixture was stirred at room temperature for 1 hour, then 12.4 g of 4,4′-diaminodiphenyl sulfone was added and the reaction proceeded as described in Example 1, giving 16.0 g of the desired product as colorless crystals, mp 227-229°C.

Example 3

2,2′-[Sulfonylbis(p-phenylenenitrilo)]bis[1-methylpyrrolidine]

To a solution of 14.9 g of N-methylpyrrolidinone in 100 ml of dry acetonitrile was added 18.4 g of phosphorus oxychloride at a temperature of 5° to 10°C. This mixture was stirred at room temperature for 1 hour, then 12.4 g of 4,4′-diaminodiphenyl sulfone was added and the reaction proceeded as described in Example 1, giving 11.4 g of the desired product as colorless crystals, mp 180-183°C.

Example 4

N′-[4-[(4-Fluorophenyl)sulfonyl]phenyl]-N,N-dimethylpropanimidamide

A mixture comprised of 38.4 g of 4-fluorothiophenol, 47.1 g of p-chloronitrobenzene, 39 g of sodium carbonate, 120 ml of water and 150 ml of ethanol was stirred at 100°C for 19 hours and then poured into 500 ml of water. The solid was collected, washed with water and recystallized from ethanol, giving 66.4 g of 4-fluoro-4′-nitrodiphenyl sulfide. A 30 g portion of this compound was added to a mixture of 200 ml of glacial acetic acid and 50 ml of 30% hydrogen peroxide and stirred at 100°C for 1 hour. The mixture was cooled, 15 ml of water was added and this mixture refrigerated overnight. The crystals were collected, giving 32.7 g of 4-fluoro-4′-nitrodiphenyl sulfone. A mixture of 10.0 g of this sulfone, 200 ml of p-dioxane and 3 ml of Raney nickel catalyst in water was hydrogenated in a Parr apparatus until hydrogen uptake ceased. The catalyst was removed by filtration. The filtrate was concentrated in vacuo and the residue crystallized from 200 ml of ethanol, giving 7.8 g of 4-[(4-fluorophenyl)sulfonyl]aniline as colorless crystals, mp 206-208°C.

To a solution of 7.07 g of N,N-dimethylpropionamide in 90 ml of dry acetonitrile was added dropwise, at 0°C to 5°C, 7.67 g (4.58 ml) of phosphorus oxychloride. After stirring for 1 hour at room temperature, 17.05 g of 4-[(4-fluorophenyl)sulfonyl]aniline was added. The resulting solution was stirred at room temperature for 4 hours, then at 60°C for 2 hours, poured into 1 liter of ice water and basified with 5N sodium hydroxide. The precipitate was collected, washed with water and dried, giving 13.13 g of desired product as a colorless solid, mp 88-90°C.

Example 5

N′-[4-[(4-Fluorophenyl)sulfonyl]phenyl-N,N-dimethylpropanimidamide, monohydrochloride

A 6.64 g portion of N′-[4-[(4-fluorophenyl)sulfonyl]phenyl-N,N-dimethylpropanimidamide in 250 ml of ether was filtered through celite. The filtrate was treated with 4 ml of 6N hydrochloric acid in isopropanol with stirring and cooling for 48 hours. The resulting solid was collected, washed with ether and dried. A 3 g portion of this solid was crystallized from 25 ml of isopropanol, giving 2.3 g of the desired product as colorless grains, mp 164-167°C.

Example 6

N′-[4-[(4-Fluorophenyl)sulfonyl]phenyl]-N,N-dimethylethanimidamide

A mixture of 2.51 g of 4-[(4-fluorophenyl)sulfonyl]aniline and 11 ml of dimethyl acetal of N,N-dimethylacetamide was stirred at 100° C. for 1 hour and then evaporated in vacuo. The residue was dissolved in a small amount of toluene and refrigerated. The precipitate was collected, giving 1.16 g of desired product, mp 115-116° C.

## Example 7

### N′-[4-[(Fluorophenyl)sulfonyl]phenyl]-N,N-dimethylethanimidamide, monohydrochloride

A 7.0 g portion of N′-[4-[(4-fluorophenyl)sulfonyl]phenyl]-N,N-dimethylethanimidamide was dissolved in a mixture of isopropanol and ether. An 8 ml portion of 6N hydrochloride acid in isopropanol was added. The resulting precipitate was collected, dissolved in a minimum amount of isopropanol and poured into a large volume of ether. The resulting white precipitate was collected, giving 6.0 g of the desired product.

## Example 8

### 3-[1-[[4-[(4-Fluorophenyl)sulfonyl]phenyl] imino]ethyl]3-azabicyclo[3.2.2]nonane

A 5.02 g portion of 3-acetyl-3-azabicyclo [3.2.2]nonane was dissolved in 40 ml of dry acetonitrile and cooled to 5°C. A 3.6 g (2.24 ml) portion of phosphorus oxychloride was then added, then the mixture was stirred at room temperature for 1 hour. A 5.02 g portion of 4-[(4-fluorophenyl)sulfonyl]aniline was added, the mixture was heated at 60°C for 2 hours and then poured into 400 ml of ice/water. The pH was adjusted to 9.0 with 5 N sodium hydroxide. The precipitate was collected, washed with water and dried. The solid was slurried in 150 ml of hexane, heated and 120 ml of chloroform added. The mixture was filtered, the solid set aside and the filtrate refrigerated overnight producing a second solid. The solids were combined, dissolved in chloroform, hexane was added and the mixture refrigerated. The resulting solid was washed with hexane and dried, giving 5.86 of the desired product.

## Example 9

### 3,3′-[Sulfonylbis(4,1-phenylenenitriloethylidyne)]bis-3-azabicyclo[3.2.2]nonane

A 3.0 g portion of 3-acetyl-3-azabicyclo [3.2.2]nonane was dissolved in 20 ml of acetonitrile. The mixture was cooled in an ice bath and 3.68 g (2.2 ml) of phosphorus oxychloride was added. The mixture was stirred for 1 hour at room temperature, 1.98 g of 4,4′-diaminodiphenyl sulfone was added and the mixture stirred at room temperature for 4 hours, then at 60°C for 2 hours. The mixture was poured into 200 ml of ice/water, the pH adjusted to 10.0 with 5N sodium hydroxide and refrigerated overnight. The pH was readjusted to 10.0 and the solid was collected, washed with water and dried. This solid was recrystallized from methylcellosolve, then slurred in water, basified to pH 12 and the product collected giving 134 mg.

## Example 10

N,′N‴-(Sulfonyldi-1,4-phenylene)bis[N,N-dimethylmethanimidamide]

A mixture of 5.0 g of 4-aminophenylsulfone and 25 ml of N,N-dimethylformamide dimethyl acetal was stirred at reflux temperature for 16 hours. The reaction solution was taken to dryness in vacuo and recrystallized from 50 ml of acetonitrile, giving 3.4 g of the desired product, mp. 147-148°C.

Example 11

N′,N‴-(Thiodi-1,4-phenylene)bis[N,N-dimethylmethanimidamide], dihydrochloride

The title compound was prepared by the procedure of Example 10 using N,N-dimethylformamide dimethyl acetal and 4-aminophenyl sulfide to yield 5.6 g of the desired compound, mp. 290-293°C.

Example 12

N′,N‴-(Sulfonyldi-1,4-phenylene)bis[N,N-dimethylethanimidamide]

A mixture of 10.0 g of 4-aminophenyl sulfone and 50 ml of N,N-dimethylacetamide dimethyl acetal was stirred at reflux temperature for 16 hours. The reac tion solution was taken to dryness in vacuo and recrystallized from acetonitrile, giving 3.7 g of product, mp. 234-236°C.

Example 13

N,N′-(Sulfonyldi-4,1-phenylene)bisethanimidic acid, diethyl ester

A mixture of 4-aminophenyl sulfone, excess triethyl orthoformate and a trace of p-toluenesulfonic acid was heated at reflux temperature for 16 hours. The excess triethyl orthoformate was removed in vacuo and the residue was used without further purification.

Example 14

1,1′-[Sulfonylbis(p-phenylene-nitrilomethylidyne)] bis[4-methylpiperazine], hydrochloride (1:x)

The title compound was prepared by the procedure of Example 12 using the product from Example 13 and 1-methylpiperazine to yield 5.4 g of tan powder, mp. 200-206°C.

Example 15

N′,N‴-(Sulfonyldi-p-phenylene)bis[N-(p-dimethylaminophenyl)methanimidamide]

The title compound was prepared by the procedure of Example 12 using the product from Example 13 and N,N-dimethyl-1,4-phenylenediamine to yield 1.1 g of yellow-green powder, mp. 210-215°C.

Example 16

1,1′-[Sulfonylbis(p-phenylene-nitrilomethylidyne)] bis[4-(2-pyridyl)piperazine]

The title compound was prepared by the procedure of Example 12 using the product from Example 13 and 1-(2-pyridyl)piperazine to yield 6.9 g of product, mp. 206-208°C.

Example 17

N′,N‴-(Sulfonyldi-p-phenylene)bis[N,N-diethylethanimidamide]

The title compound was prepared by the procedure of Example 1 using 4-aminophenyl sulfone and N,N-diethylacetamide to yield 14.7 g of product, mp. 222-224°C.

Example 18

N′,N‴-[Sulfonylbis(6-fluoro-3,1-phenylene)] bis[N,N-dimethylmethanimidamide]

The title compound was prepared by the procedure of Example 12 using 3-amino-4-fluorophenyl sulfone and N,N-dimethylformamide dimethyl acetal to yield 8.0 g of product as an oil.

Example 19

N′,N‴-(Sulfonyldi-4,1-phenylene)bis[N,N-diethylbenzenecarboximidamide]

The title compound was prepared by the procedure of Example 1 using 4-aminophenyl sulfone and N,N-diethylbenzamide to yield 12.1 g of product, mp. 167-169°C.

Example 20

N′,N‴-(Sulfonyldi-4,1-phenylene)bis[N,N-diethylpropanimidamide], dihydrochloride

The title compound was prepared by the procedure of Example 1 using 4-aminophenylsulfone and N,N-diethylpropionamide to yield 45.0 g of colorless crystals, mp. 174-176°C. The hydrochloride salt, prepared with dichloromethane and hydrogen chloride gas, was a colorless glass.

Example 21

N′,N‴-(Sulfonyldi-4,1-phenylene)bis[N,N-dimethylpentanimidamide], dihydrochloride

The title compound was prepared by the procedure of Example 1 using 4-aminophenyl sulfone and N,N-dimethylvaleramide. The off-white powder was dissolved in dichloromethane, hydrogen chloride gas was bubbled through the solution and the dihydrochloride was precipitated as a colorless glass.

Example 22

4,4′-Sulfonylbis[N-(1,3-dimethyl-2-imidazolidinylidene)benzenamine]

The title compound was prepared by the procedure of Example 1 using 4-aminophenyl sulfone and 1,3-dimethylimidazolidinone to yield 6.0 g of colorless crystals, mp. 91-92°C.

Example 23

N′,N‴-(Sulfonyldi-4,1-phenylene)bis[N-methyl-N-phenylethanimidamide]

The title compound was prepared by the procedure of Example 1 using 4-aminophenyl sulfone and N-methylacetanilide to yield 8.3 g of colorless crystals, mp. 92-93°C.

Example 24

N′,N‴-(Sulfonyldi-3,1-phenylene)bis[N,N-dimethylethanimidamide]

The title compound was prepared by the procedure of Example 1 using 3-aminophenyl sulfone and N,N-dimethylacetamide to yield 1.2 g of a brown powder.

Example 25

N′,N‴-(Sulfonyldi-4,1-phenylene)bis[N,N-diethyl-4-(trifluoromethyl)benzenecarboximidamide]

The title compound was prepared by the procedure of Example 1 using 4-aminophenyl sulfone and

N,N-diethyl-4-(trifluoromethyl)benzamide, to yield 0.845 g of product, mp. 196-198°C.

Example 26

N,N-Diethyl-3-thiophenecarboxamide

To a 0°C nitrogen flushed solution of 7.5 g of thiophene 3-carboxylic acid, 6.44 ml of N-methylmorpholine, and 150 ml of tetrahydrofuran was added 7.3 ml of trimethylacetyl chloride. The mixture was stirred at 0°C for 1 hour followed by the addition of 6.04 ml of diethylamine in 40 ml of tetrahydrofuran. The reaction was stirred at room temperature for 16 hours, concentrated in vacuo and dissolved in dichloromethane. The organic layer was washed with 10% NaOH, 10% HCl, water and a saturated brine solution. The dichloromethane solution was dried over sodium sulfate, concentrated in vacuo and purified by column chromatography giving 6.59 g of a yellow oil.

Example 27

N′,N‴-(Sulfonyldi-4,1-phenylene)bis[N,N-diethyl-3-thiophenecarboximidamide]

The title compound was prepared by the procedure of Example 1 using N,N-diethyl-3-thiophenecarboxamide and 4-aminophenyl sulfone to yield 7.1 g of product, mp. 135-140°C.

Example 28

1,1′-[Sulfonylbis[4,1-phenylenenitrilo(phenylmethylidyne)]]bis[4-methylpiperazine]

The title compound was prepared by the procedure of Example 1 using 4-aminophenyl sulfone and 1-benzoyl-4-methylpiperazine to yield 3.0 g of product, mp. 210-212°C.

Example 29

1,1′-[Sulfonylbis[4,1-phenylenenitrilo[(4-fluorophenyl)methylidyne]]]bis[4-(4-fluorophenyl) piperazine]

The title compound was prepared by the procedure of Example 1 using 4-aminophenyl sulfone and 1-(4-fluorobenzoyl)-4-(4-fluorophenyl)piperazine to yield 2.1 g of product, mp. 249-251°C.

Example 30

N′,N‴-(Sulfonyldi-3,1-phenylene)bis[N,N diethylethanimidamide]

The title compound was prepared by the procedure of Example 1 using 3-aminophenyl sulfone and N,N-diethylacetamide to yield 2.0 g of pale brown powder.

Example 31

N′,N‴-(Sulfonyldi-4,1-phenylene)bis[N-ethylethanimidamide]

The title compound was prepared by the procedure of Example 1 using 4-aminophenyl sulfone and N-ethylacetamide to yield 1.0 g of pale yellow glass.

Example 32

N′,N‴-(Sulfonyldi-4,1-phenylene)bis[N,N-diethyl-3-pyridinecarboximidamide]

The title compound was prepared by the procedure of Example 1 using 4-aminophenyl sulfone and N,N-diethylnicotinamide to yield 12.4 g of off-white powder.

Example 33

N,N-Diethyl-4-fluoro-N′-[4-[(4-fluorophenyl) sulfonyl]phenylbenzenecarboximidamide]

The title compound was prepared by the procedure of Example 1 using N,N-diethyl-4-(fluoro)benzamide and p-(p-fluorophenylsulfonyl)aniline to yield 3.0 g of product, mp. 149-151°C.

Example 34

N′,N‴-(Sulfonyldi-3,1-phenylene)bis [N,N-dimethylmethanimidamide]

The title compound was prepared by the procedure of Example 1 using 3-aminophenyl sulfone and N,N-dimethylacetamide to yield 5.2 g of product.

Example 35

N′,N‴-(Sulfonyldi-3,1-phenylene)bis [N,N-diethylpropanimidamide]

The title compound was prepared by the procedure of Example 1 using 3-aminophenyl sulfone and N,N-diethylpropionamide to yield the product as a brown semi-solid.

Example 36

N′,N‴-(Sulfonyldi-3,1-phenylene)bis [N-methylpropanimidamide]

The title compound was prepared by the procedure of Example 1 using 3-aminophenyl sulfone and N-methylpropionamide to yield the product as a brown gum.

Example 37

N′,N‴-(Sulfonyldi-3,1-phenylene)bis [N,N-diethylmethanimidamide]

The title compound was prepared by the procedure of Example 1 using 3-aminophenyl sulfone and N,N-diethylformamide to yield 7.2 g of a brown oil.

Example 38

N′,N‴-(Sulfonyldi-3,1-phenylene)bis[N,N-diethyl-3-pyridinecarboximidamide]

The title compound was prepared by the procedure of Example 1 using 3-aminophenyl sulfone and N,N-diethylnicotinamide to yield 11.2 g of a brown semi-solid.

Example 39

1-[[[3-[(2-Aminophenyl)sulfonyl]phenyl]imino] cyclopropylmethyl]-piperidine

The title compound was prepared by the procedure of Example 1 using 2,3′-sulfonyldianiline and cyclopropylcarboxylpiperide to yield the product as a yellow gum.

Example 40

N-(1-Butyl-2-pyrrolidinylidene)-2-[[4-[(1-butyl-2-pyrrolidinylidene)amino]phenyl]sulfonyl]-benzenamine

The title compound was prepared by the procedure of Example 1 using 2,4′-sulfonyldianiline and N-n-butyl-2-pyrrolidone to yield 0.80 g of the product as a pink oil.

Example 41

N′-[2-[[3-[[(Dimethylamino)methylene]amino]phenyl]sulfonyl]phenyl]-N,N-dimethyl-methanimidamide

To a nitrogen flushed solution of 137.4 mg (145 μl) of N,N-dimethyl formamide in 1 ml of dry acetonitrile was added 230 mg (140 μl) of phosphorsus oxychloride. The solution was stirred at room temperature of 1.5 hours, 248.3 mg of 2,3′-sulfonyldianiline was added and the reaction was stirred for 16 hours at 45°C. The reaction was diluted with ice water, made basic with 5N sodium hydroxide and the desired product collected, mp 124-126°C.

## Claims

1. A compound selected from the group consisting of those of the formula:

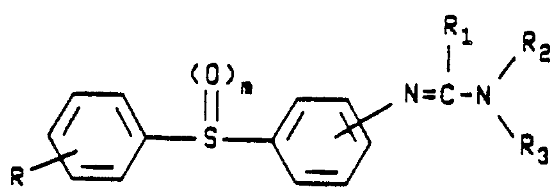

wherein m is 0, 1 or 2; R is hydrogen, amino, halogen, or a moiety of the formula:

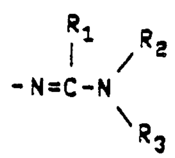
,

$R_1$ is hydrogen, alkyl($C_1$-$C_4$), pyridine, phenyl,

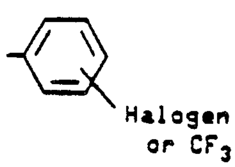
,

or thienyl, with the proviso that when $R_1$ is hydrogen, both $R_2$ and $R_3$ may not be hydrogen or lower alkyl, or $R_2$ and $R_3$ taken together with the associated nitrogen may not be pyrrolidino, piperidino or morpholino, and when $R_1$ is phenyl, both $R_2$ and $R_3$ may not be hydrogen;
$R_2$ is hydrogen or alkyl($C_1$-$C_4$);
$R_3$ is hydrogen, alkyl($C_1$-$C_4$), phenyl or N,N-dimethylaniline;
$R_1$ and $R_2$ taken together is $-(CH_2)_n-$ wherein n is an integer from 2 to 4 or $R_1$ and $R_2$ taken together form a moiety of the formula;

$-\ \underset{\displaystyle CH_3}{\overset{}{N}} - CH_2CH_2-$

where $R_3$ is $CH_3$;
$R_2$ and $R_3$ taken together with the associated nitrogen is pyrrolidino, piperidino, morpholino, thiomorpholino, 4-methylpiperazino, 3-azabicyclo [3.2.2]nonyl, 2-pyridylpiperazino or 4-fluorophenylpiperazino; and the pharmaceutically acceptable salts thereof.

2. The compound according to claim 1 wherein m is 2.

3. The compound according to claim 1 wherein m is 0, 1 or 2; $R_1$ is alkyl($C_1$-$C_4$); $R_2$ is hydrogen or alkyl($C_1$-$C_4$) and $R_3$ is hydrogen, alkyl($C_1$-$C_4$), phenyl or N,N-dimethylaniline and R is as recited in claim 1.

4. The compound according to claim 1 wherein m is 0, 1 or 2, $R_1$ is pyridine, phenyl,

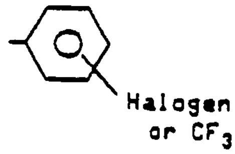

,

or thienyl; and R, $R_2$ and $R_3$ are as recited in claim 1.

5. The compound according to claim 1 wherein m is 0, 1 or 2, R is as recited in claim 1; $R_1$ is alkyl($C_1$-$C_4$), pyridine, thienyl, phenyl or

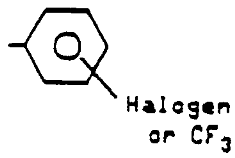

;

$R_2$ and $R_3$ taken together with the associated nitrogen is pyrrolidino, piperidino, 4-methylpiperazino, 2-pyridylpiperazino or 4-fluorophenylpiperazino.

6. The compound according to claim 1 wherein m and R are as recited in claim 1, $R_1$ is alkyl($C_1$-$C_4$), pyridine, phenyl, thienyl or

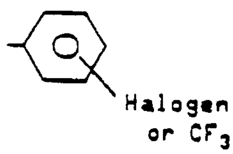

;

and $R_2$ and $R_3$ taken with the associated nitrogen is morpholino or thiomorpholino.

7. The compound according to claim 1 wherein m is 0,1 or 2, R is fluoro or a moiety of the formula;

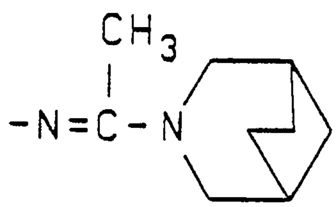

,

$R_1$ is methyl and $R_2$ and $R_3$ together with the associated nitrogen form a moiety of the formula;

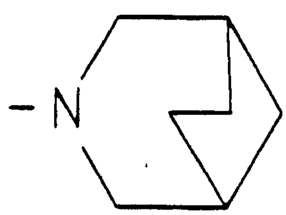

.

8. The compound according to claim 1;
N′,N‴-(sulfonyldi-p-phenylene)bis[N,N-diethylbutyramidine].

9. The compound according to claim 1;
N′,N‴-(sulfonyldi-p-phenylene)bis[N,N-dimethylpropionamidine].

10. The compound according to claim 1;
2,2′-[sulfonylbis(p-phenylenenitrilo)bis[1-methylpyrrolidine.

11. The compound according to claim 1;

N′-[4-[(4-fluorophenyl)sulfonyl]phenyl]-N,N-dimethylpropanimidamide.

12. The compound according to claim 1; N′-[4-[(4-fluorophenyl)sulfonyl]phenyl]-N,N-dimethylpropanimidamide, monohydrochloride.

13. The compound according to claim 1; N′-[4-[(4-fluorophenyl)sulfonyl]phenyl]-N,N-dimethylethanimidamide.

14. The compound according to claim 1; N′-[4-[(4-fluorophenyl)sulfonyl]phenyl]-N,N,-dimethylethanimidamide, monohydrochloride.

15. The compound according to claim 1; 3-[1-[[4-[(4-fluorophenyl)sulfonyl]phenyl]imino]ethyl]-3-azabicyclo[3.2.2]nonane.

16. The compound according to claim 1; 3,3′-[sulfonylbis(4,1-phenylenenitriloethylidyne)]bis-3-azabicyclo[3.2.2]nonane.

17. The compound according to claim 1; N′,N‴-(sulfonyldi-1,4-phenylene)bis(N,N-dimethylethanimidamide).

18. The compound according to claim 1; N′,N‴-(sulfonyldi-p-phenylene)bis[N,N-diethylethanimidamide].

19. The compound according to claim 1; N,N-diethyl-4-fluoro-N′-[4-[(4-fluorophenyl)sulfonyl]phenylbenzene carboximidamide].

20. The compound according to claim 1; 1,1′-[sulfonylbis[4,1-phenylenenitrilo[(4-fluorophenyl)methylidyne]]]bis[4-(4-fluorophenyl)piperazine].

21. The compound according to claim 1; N.N‴-(Sulfonyldi-4,1-phenylene)bis[N,N-diethyl-3-pyridine carboximidamide).

22. The compound according to claim 1; N′,N‴-[Sulfonylbis(6-fluoro-3,1-phenylene)]bis[N,N-dimethylmethanimidamide]

23. The compound according to claim 1; N′,N‴-(Sulfonyldi-4,1-phenylene)bis[N,N-diethylbenzenecarboximidamide].

24. The compounds according to claim 1; N′,N‴-(Sulfonyldi-4,1-phenylene)bis[N,N-diethylpropanimidamide] and the hydrochloride salt thereof.

25. The compound according to claim 1; N′,N‴-(sulfonyldi-3,1-phenylene)bis[N,N-dimethylethanimidamide].

26. The compound according to claim 1; 4,4′-sulfonylbis[N-(1,3-dimethyl-2-imidazolidinyldene)benzenamine.

27. A method of modulating the immune response system in a warm-blooded animal which comprises administering to said animal an immune response modulating amount of a compound of the formula:

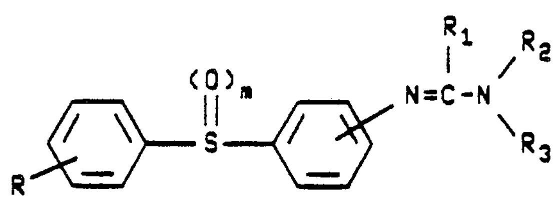

wherein m is 0, 1 or 2;

R is hydrogen, halogen, amino or a moiety of the formula;

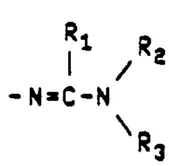
,

$R_1$ is hydrogen, alkyl($C_1$-$C_4$), pyridine, phenyl,

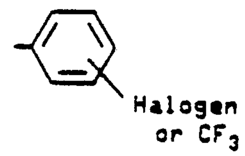

,

or thienyl; $R_2$ is hydrogen or alkyl($C_1$-$C_4$); $R_3$ is hydrogen, alkyl($C_1$-$C_4$), phenyl or N,N-dimethylaniline; $R_1$ and $R_2$ taken together is $-(CH_2)_n-$ wherein n is an integer from 2 to 4 or $R_1$ and $R_2$ taken together form a moiety of the formula

$-N-CH_2CH_2-$
$\ \ |$
$\ \ CH_3$

where $R_3$ is $CH_3$; $R_2$ and $R_3$ taken together with the associated nitrogen is pyrrolidino, piperidino, morpholino, thiomorpholino, 4-methylpiperazino, 3-azabicyclo[3.2.2]nonyl, 2-pyridylpiperazino or 4-fluorophenylpiperazino; and the pharmaceutically acceptable salts thereof.

28. A method of stimulating the proliferation and differentiation of blood cell progenitors in bone marrow of warm-blooded animals which comprises administering to said animals a therapeutically effective amount of a compound according to claim 27.

29. A method of accelerating the recovery of white blood cell progenitors in bone marrow of warm blooded animals when used in conjunction with chemical or irradiation therapy which comprises administering to said animals a therapeutically effective amount of a compound according to claim 27.

30. A method of accelerating the recovery of white blood cell progenitors in bone marrow of warm-blooded animals following chemical therapy which comprises administering to said animals a therapeutically effective amount of a compound according to claim 27.

31. A method of accelerating the recovery of white blood cell progenitors in bone marrow of warm blooded animals when given prior to irradiation therapy which comprises administering to said animals a therapeutically effective amount of a compound according to claim 27.

32. A method of enhancing the activity of immune cells which inhibit tumor growth in warm-blooded animals which comprises administering to said animals a therapeutically effective amount of a compound according to claim 27.

33. A method of enhancing the activity of immunoregulatory proteins which inhibit tumor growth in warm-blooded animals which comprises administering to said animals a therapeutically effective amount of a compound according to claim 27.

34. A method of enhancing the activity of immune cells and immunoregulatory proteins which inhibit bacterial growth in warm-blooded animals which comprises administering to said animals a therapeutically effective amount of a compound according to claim 27.

35. A method of enhancing the activity of immune cells and immunoregulatory proteins which inhibit viral growth in warm-blooded animals which comprises administering to said animals a therapeutically effective amount of a compound according to claim 27.

36. A pharmaceutical composition comprising an immune response modulating amount of a compound according to claim 1 in association with a pharmaceutically acceptable carrier, diluent or excipient.

37. A composition according to claim 35 in unit dosage form.

38. The process for preparing compounds of the formula;

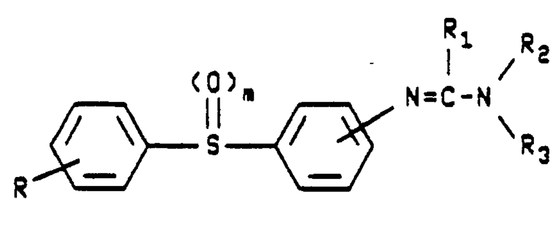

wherein R is hydrogen, halogen, amino or a moiety of the formula;

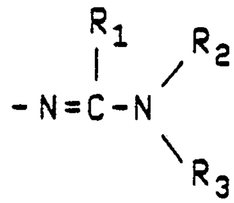

,

m is 0, 1 or 2;

$R_1$ is hydrogen, alkyl($C_1$-$C_4$), pyridine, phenyl,

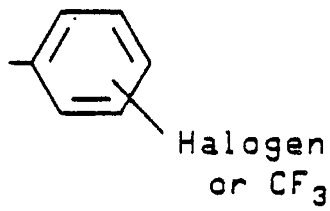

,

or thienyl; with the proviso that when $R_1$ is hydrogen, both $R_2$ and $R_3$ may not be hydrogen or lower alkyl or $R_2$ and $R_3$ taken together with the associated nitrogen may not be pyrrolidino, piperidino or morpholino, and when $R_1$ is phenyl, both $R_2$ and $R_3$ may not be hydrogen;

$R_2$ is hydrogen or alkyl($C_1$-$C_4$);

$R_3$ is hydrogen, alkyl($C_1$-$C_4$), phenyl or N,N-dimethylaniline;

$R_1$ and $R_2$ taken together is $-(CH_2)_n-$ wherein n is an integer from 2 to 4 or $R_1$ and $R_2$ taken together form a moiety of the formula;

$- \underset{CH_3}{\underset{|}{N}} - CH_2CH_2-$

where $R_3$ is $CH_3$; $R_2$ and $R_3$ taken together with the associated nitrogen is pyrrolidino, piperidino, morpholino, thiomorpholino, 4-methylpiperazino, 3-azabicyclo[3.2.2]nonyl, 2-pyridylpiperazino or 4-fluorophenylpiperazino; and the pharmaceutically acceptable salts thereof, which comprises treating an amide of the formula;

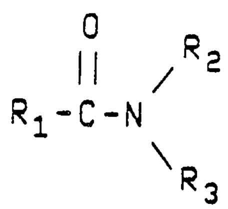

,

wherein $R_1$, $R_2$ and $R_3$ are as hereinbefore defined with phosphorus oxychloride in acetonitrile at about 0-10°C, and then adding a hemimolar amount of 4-aminophenylsulfone or an equimolar amount of 4-(4-halophenylsulfonyl) aniline and stirring the resulting reaction mixture followed by separation of the desired product from the reaction mixture.

Figure 1

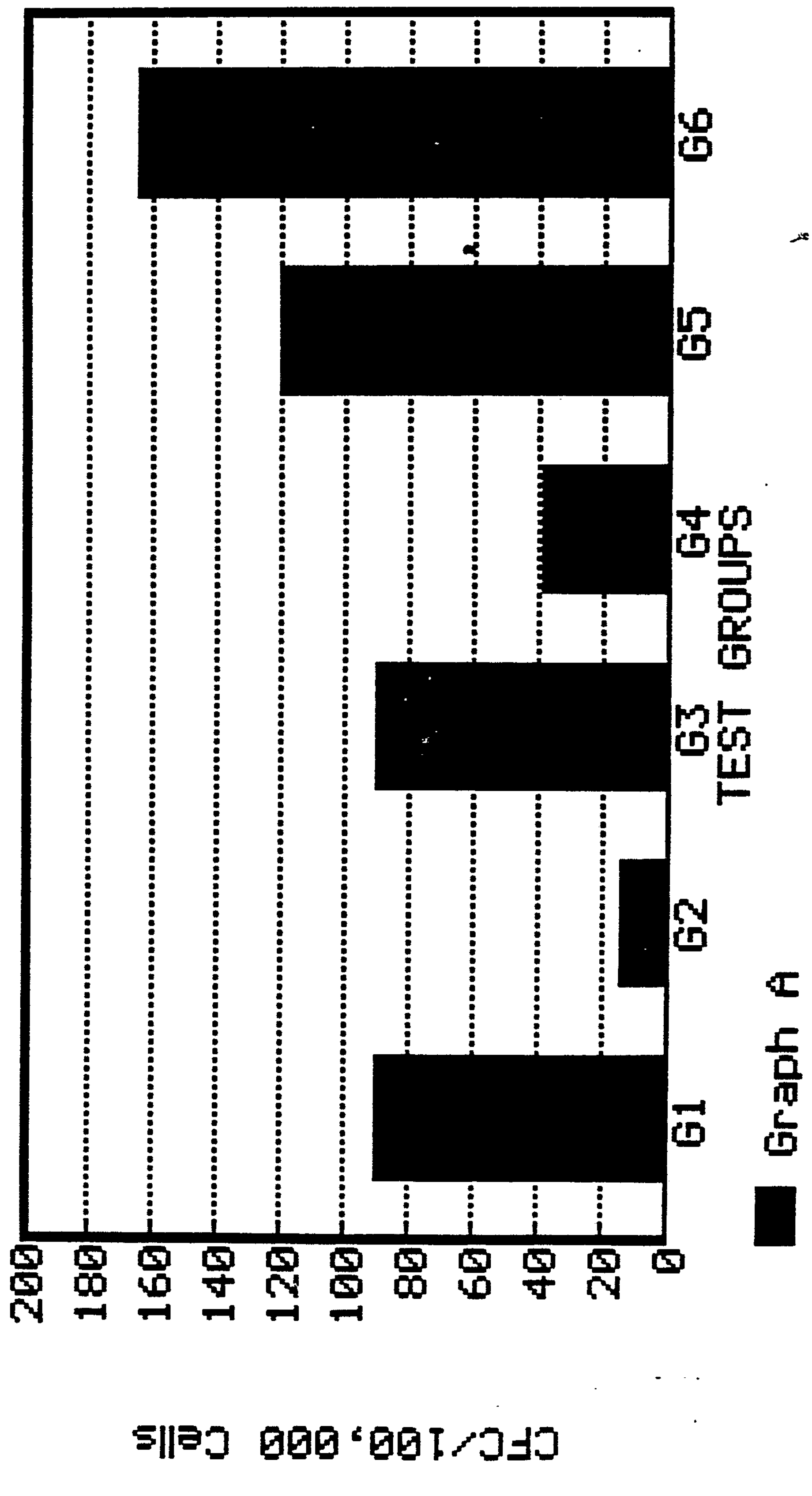
CFC/100,000 Cells
200
180
160
140
120
100
80
60
40
20
0
G1
G2
G3
G4
G5
G6
TEST GROUPS
Graph A

Figure 2

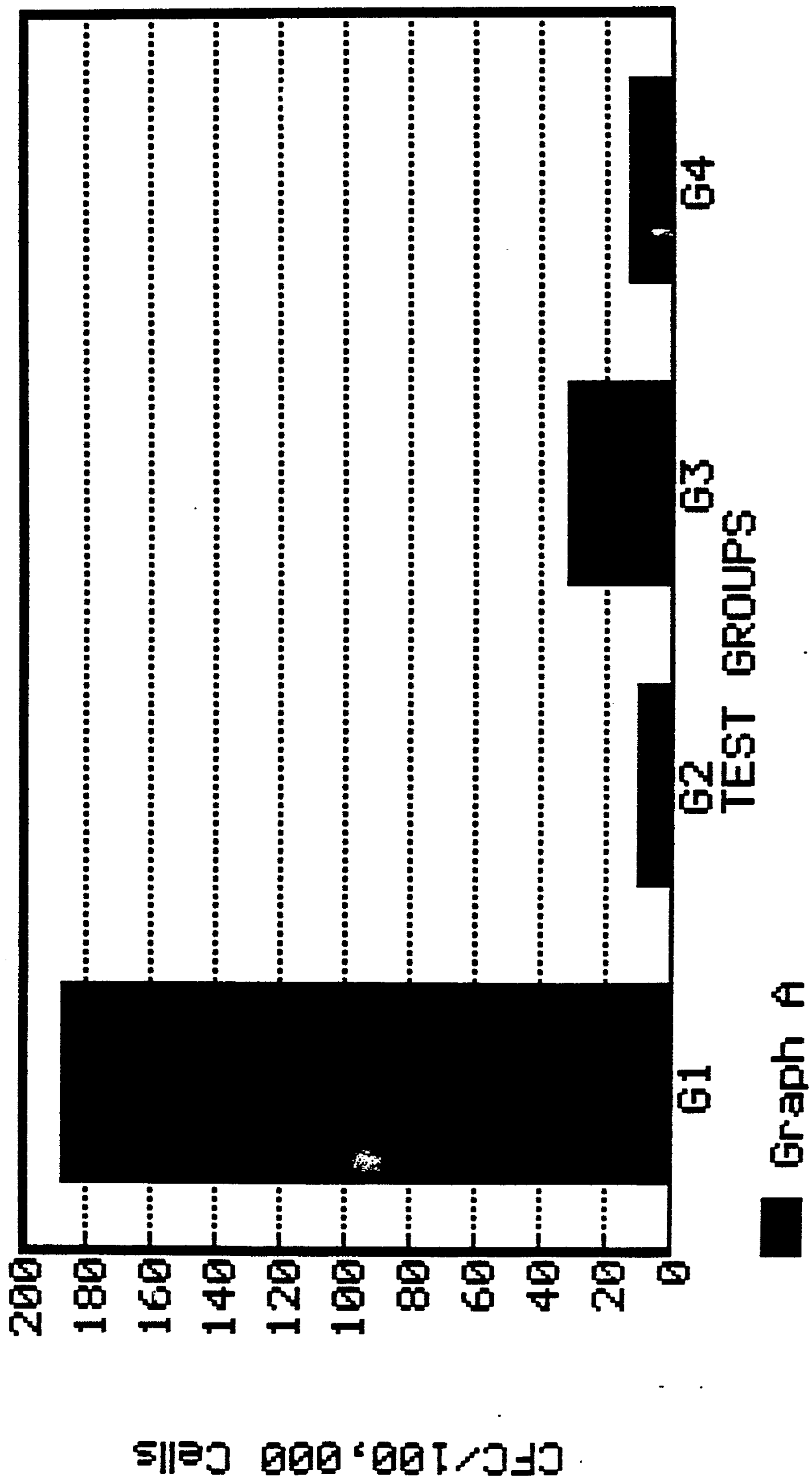
CFC/100,000 Cells
200
180
160
140
120
100
80
60
40
20
0
G1
G2
G3
G4
TEST GROUPS
Graph A

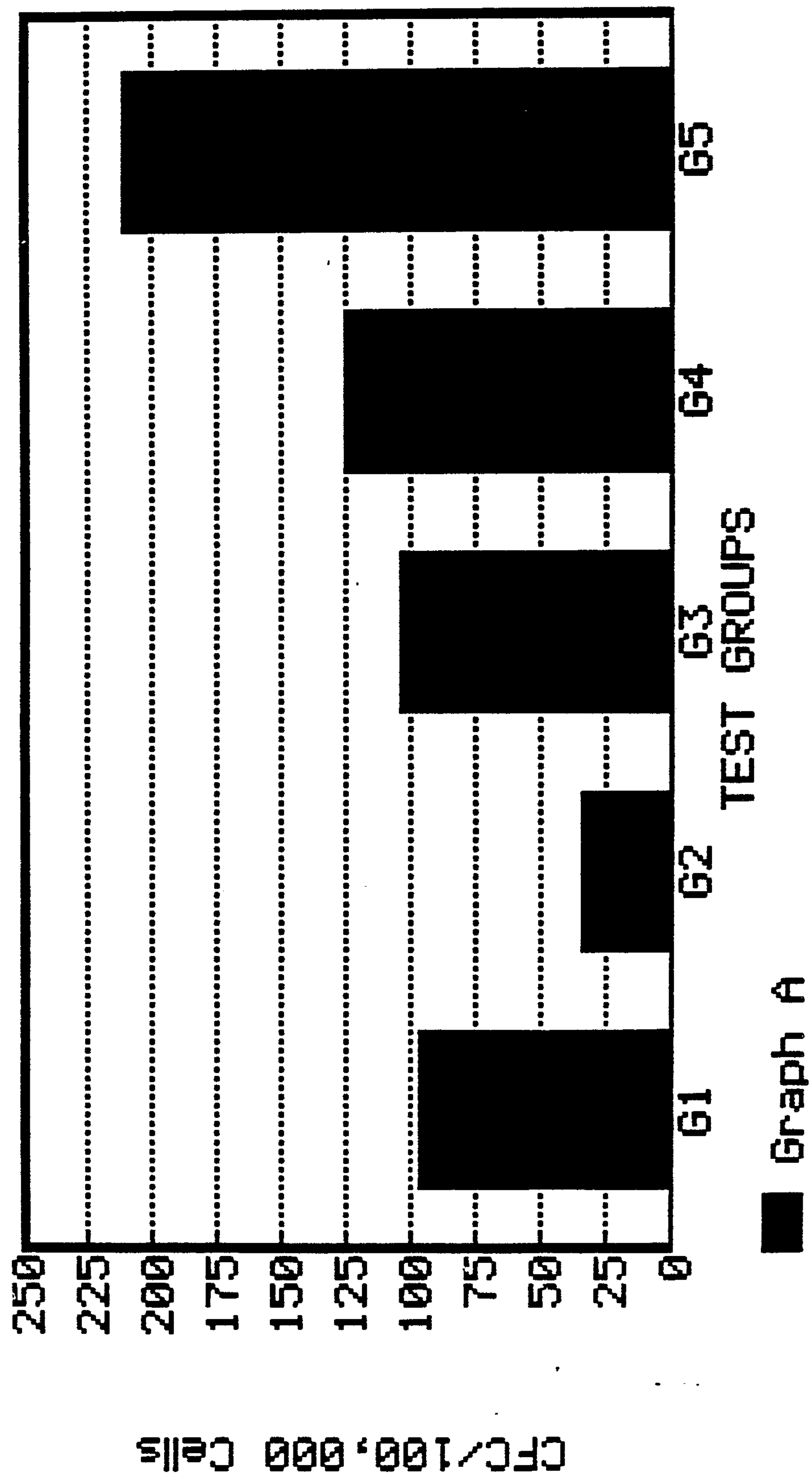

CFC/100,000 Cells
250
225
200
175
150
125
100
75
50
25
0
G1
G2
G3
G4
G5
TEST GROUPS
Graph A


Figure 3

Figure 4

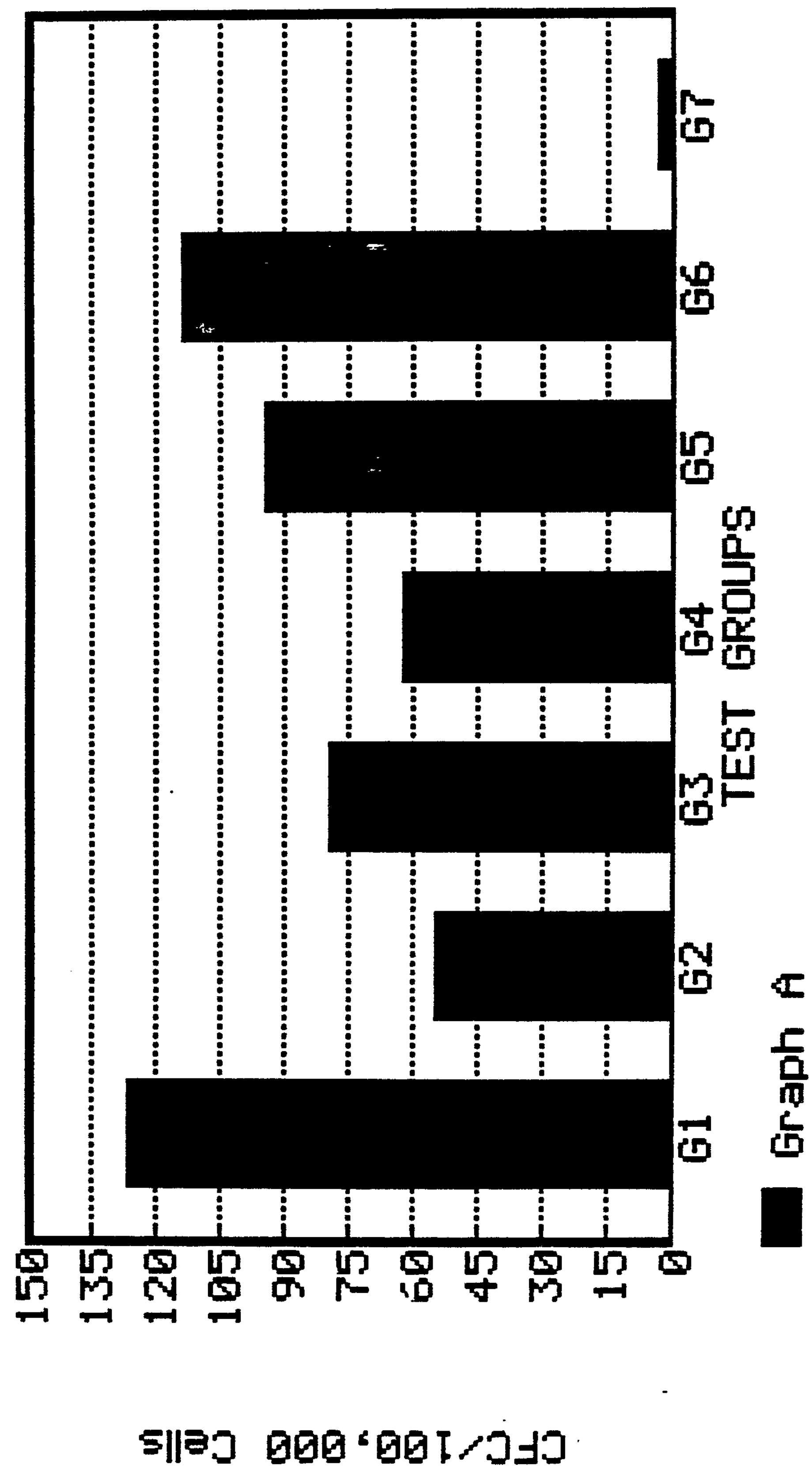
CFC/100,000 Cells
150
135
120
105
90
75
60
45
30
15
0
G1
G2
G3
G4
G5
G6
G7
TEST GROUPS
Graph A

European Patent Office

# EUROPEAN SEARCH REPORT

Application number EP 89107998.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 1 353 520 (MERCK & CO INC) * Claims 1,24,28 * -- | 1,36, 38 | C 07 C 147/12<br>C 07 C 149/42<br>C 07 D 295/14<br>C 07 D 333/24<br>A 61 K 31/155 |
| A | DE - A1 - 3 419 009 (DR. KRAL THOMAE) * Claims 1,7,10 * ---- | 1,36, 38 | |

| TECHNICAL FIELDS SEARCHED (Int. Cl.4) |
|---|
| C 07 C 147/00<br>C 07 C 149/00<br>C 07 D 295/00<br>C 07 D 333/00 |

The present search report has been drawn up for all claims

| Place of search | Date of completion of the search | Examiner |
|---|---|---|
| VIENNA | 26-07-1989 | REIF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82